# EUROPEAN PATENT APPLICATION

(11) **EP 4 403 027 A1**
(43) Date of publication of application: **24.07.2024**
(21) Application number: 23152241.8
(22) Date of filing: 18.01.2023
(51) Int. Cl.: A01K 29/00, A61B 5/107, G01B 11/24, G06V 10/10

(54) **SCANNING SYSTEM AND SCANNING METHOD FOR RECORDING ANIMAL MEASUREMENTS**

(71) Applicant: YOUDOME SARL, 98000 Monaco (MC)
(72) Inventor: BOCQUET, Xavier, 98000 MONACO (MC); BARAT, Christian, 06400 CANNES (FR); DE MARTIS, Riccardo, 09134 CAGLIARI (IT); LAINARD, Fabrice, 17550 DOLUS (FR)
(74) Representative: Axe PI

(57) **Abstract**

The present invention relates to a scanning device (2) for recording quadruped animal measurements, comprising two frames (3) parallel and symmetrically spaced on both sides of a scanning area (4) dimensioned to allow an animal (1) inside, a scanning means (5), comprising at least two cameras (50) mounted on each of the two frames (3), and having an acquisition scope orientated toward the scanning area (4), a digital storage means (6) for storing the images and/or the videos captured by the cameras (50), characterized in that the cameras (50) being of a type of depth camera, and in that the scanning means (5) comprise at least six cameras (50) grouped in three pairs, each of the three pairs of the at least six cameras (50) being symmetrically mounted on the at least two frames (3).

The invention also relates to a scanning system and a scanning method.

## Description

### Technical Field

The present invention relates to a scanning device, a scanning system and a scanning method for recording animal measurements.

The invention relates to the technical field of animal husbandry and breeding.

In particular, the invention is dedicated to recording measurements of animals, especially of quadruped animals. Such animals can be of any species, especially livestock animals such as sheep and cattle (*i.e*. oxen and cows), goats, eventually deer, dogs and cats, and preferably horses.

Such animal measurements are usually performed to evaluate the animal anatomy and morphology, in order to determine health, breeding and also performance data, which are useful to breeding and veterinary surveys of the animals. Such data are also used to confirm and ensure a pedigree of an animal.

Moreover, the animal measurements can be used to produce and manufacture customized horse equipment, such as a saddle or a harness, specifically dedicated to suiting each of the horses scanned by the invention.

The animal measurements can also be used for digital identification and certification of an animal, for example for the creation of biometric passport, also for media promotion.

Nowadays pedigree and breeding data of animals, such as horses, are managed by dedicated organizations, providing individual identification data of each animal. Such data can include many types of individual information relative to the animal, such as measurements of different body parts, weight, eye color, skin or fur color, but also biological information, for example blood type.

Moreover, the individual identification of an animal involves different marking means and methods, such as ear tags, tattoos, retina scans, subcutaneous contactless microchips (*e.g*. using RFID for « Radio Frequency IDentification » and/or NFC for « Near Field Communication). Once the animal is identified, all the previously recorded data are accessible, through well-known computer and communication technologies.

The animal identification data are useful in related applications, such as breeding, but also for selling animals, for example through commercial auctions.

Therefore, it is primordial to precisely collect and record animal information, especially animal measurements, in order to characterize correctly each individual animal.

### Background Art

Currently the state of the art uses empirical methods, wherein an animal specialist, such as a vet, manually measures each animal, one by one, with a measuring stick or tape. The specialist can also use an angle blade to measure angles of different anatomical parts of the animal. The animal can also be placed on a scale, to measure its weight. Then, the measurements are compared to patterns, drawings, and diagrams, to sort and combine different anatomical characteristics of the animal, for a comparison with the ideal forms to finally individually evaluate each of the animals.

Such a manual process has several drawbacks, due to the potential errors made when measuring the animals, which should remain still at all times. This operation of measuring involves a considerable waste of time. Moreover, some manually acquired information is not precise; for example, the size and the weight can only give a globally calculated body mass index, which is not representative of the real body mass of specific body parts, such as the body fat or the body muscle (*e.g*. meat) for livestock or leg musculature for a competitive animal, such as a race horse.

Therefore, known solutions use digital techniques for contactless measuring of animals through digitally scanned images of the animals.

Moreover, considering some animal species, especially horses, a behavioral analysis of the movements, so-called « gait analysis », is required for studying and recording movement data, such as stride frequencies for walk, trot, canter and gallop.

Known solutions use complex tri-axial accelerometer measuring systems, which include various processes, like image sequencies, video graphics, speed cinematography, optoelectronic technics and the like.

The gait analysis is even more complicated when coupled with a rider, during a race or training, to collect data relative to the horse-rider interactions.

In any way, the above-mentioned known solutions tend to be incomplete, lack in accuracy, eventually invasive and cumbersome, and suffer from limitations in various aspects to obtain realistic information in natural conditions.

### Summary of Invention

An object of the present invention is to improve a scanning device and a scanning system for recording animal measurements, which provides non-contact individual animal measuring for a faster and easier, more accurate and more efficient recording of individual animals' anatomical characteristics.

Therefore, a first object of the invention relates to a scanning device for recording quadruped animal measurements, comprising
- at least two frames, each being located on a right side and on a left side, being parallel and symmetrically spaced from each other,
- a scanning area defined between the at least two frames, the scanning area being dimensioned to allow an animal inside,
- a scanning means, comprising at least two cameras being of an image type and/or of a video type,
   i) each of the at least two cameras being mounted on each of the at least two frames, on the right side and the left side,
   ii) each of the at least two cameras having an acquisition scope orientated toward the scanning area,
- a digital storage means for storing the images and/or the videos captured by the at least two cameras.

Such a scanning device is characterized in that:
- the cameras being a type of depth camera,
   and in that:
   the scanning means comprise
   - at least six cameras grouped in three pairs, each of the three pairs of the at least six cameras being symmetrically mounted on the at least two frames.

According to an embodiment, each of the at least two frames comprises
- a first extremity and an opposite extremity,
- a first tripod at the first extremity,
- a second tripod at the opposite extremity,
- a middle tripod, located between the first tripod and the second tripod,
   the at least six cameras being mounted on the corresponding first tripod, second tripod and middle tripod.

According to an embodiment, the scanning means comprise
- at least eight cameras grouped in four pairs, each of the four pairs of the at least eight cameras being symmetrically mounted on the at least two frames.

According to an embodiment, the scanning means comprise at least twenty-two cameras grouped in eleven pairs, each of the eleven pairs of the at least twenty-two cameras being symmetrically mounted on the at least two frames.

According to an embodiment,
- the first tripod of each of the at least two frames comprises five of the at least twenty-two cameras, vertically located at different first heights along the first tripod,
- the second tripod of each of the at least two frames comprises three of the at least twenty-two cameras, vertically located at different second heights along the second tripod,
- the middle tripod of each of the at least two frames comprises three of the at least twenty-two cameras, vertically located at different middle heights along the middle tripod.

According to an embodiment, the at least two frames being spaced from borders of the scanning area, so that each of the cameras is spaced with a lateral distance from a median longitudinal plane of the scanning area, the lateral distance being from 60 cm to 120 cm.

According to an embodiment, the lateral distance is approximately 80 cm, preferably at a maximum of 80 cm.

According to an embodiment, the at least two frames are spaced managing an entry of the scanning area at a first end and an output of the scanning area at an opposite end, defining a corridor between the at least two frames, the scanning area is dimensioned to allow an animal to move along the corridor, crossing the scanning area through the entry to the output.

According to an embodiment, the scanning device further comprises a calibration target, the calibration target being a black and white checkered board, movable to be placed in the scanning area along a median longitudinal plane, in front of the at least six cameras.

According to an embodiment, the at least six cameras have capture wavelengths close to infrared light, preferably capture wavelengths about 850 nanometers.

According to an embodiment, each of the at least six cameras being a type of depth camera having at least an internal sensor of a color depth RGB-D type.

The invention also relates to a scanning system for recording quadruped animal measurements, comprising
- the scanning device according to any of the preceding claims,
- software computing data, the software being installed on and executed by a computer terminal linked to the digital storage means, the data being digital and comprising at least the images and/or the videos captured by the at least six cameras, wherein
- the software comprises a conversion module of the data, the conversion module transforming the data into a tri-dimensional point cloud,
   characterized in that
   - the software comprises an extracting module of the animal measurements from the point cloud.

According to an embodiment, the conversion module transforms at least one depth map of the points into the point cloud.

According to an embodiment, the conversion module comprises at least one filter, the filter being applied to the at least one depth map of points.

The invention also relates to a scanning method for recording animal measurements, using the scanning device according to the invention, wherein
- positioning the calibration target along the median longitudinal plane of the scanning area,
- calibrating the cameras on checkered squares of the calibration target,
- removing the calibration target,
- placing an animal inside the scanning area,
- capturing animal images and/or videos with all of the cameras,
   characterized in that it comprises the steps of
   - calibrating the cameras by detecting the position of at least one corner of one of the checkered squares captured by each of the cameras,
   - calculating a global repository of all the cameras relative to each other, regarding the at least one of the corners' detected position,
      and in that
      - storing captured images and/or videos into digital data,
      - converting the digital data into a tri-dimensional point cloud in the global repository,
      - extracting the animal measurements from the point cloud.

Thus, the scanning device and system according to the invention reduce the cognitive burden on a user and produce a non-invasive means for measuring animals. They also conserve handling time and optimize animal emotional stress syndrome.

Especially, the invention allows to simplify measuring, without a specialist, such as a vet, to conduct the measurements on the animal. Thanks to an automatic digital scanning device, any owner can easily understand and use the scanning device to record measurements on his own.

The invention is preferably movable so as to be transported from a provider to a remote location, e.g. where the animal is located for recording the measurements at the husbandry site, then be sent back to the provider. Moreover, as the invention is digital and can be connected to communication networks, the data recorded can be stored directly on site and/or remotely, eventually shared to any third-party organization.

Moreover, the measurements obtained can be used to produce and manufacture customized horse equipment, such as a saddle or a harness, specifically dedicated to suiting each of the horses scanned by the invention.

The animal measurements obtained can also be used for digital identification and certification of an animal, for example for the creation of biometric passport, also for media promotion.

### Brief description of the Drawings

The invention can be better understood on reading the following description given merely by way of example and with reference to the accompanying drawings, in which:
FIG. 1 is a diagrammatical lateral view of an embodiment of the scanning device, with a horse-type animal inside the scanning area on the ground, especially showing eleven cameras of the scanning means, with five cameras in front of the horse, and also dedicated vertical angular orientations of the cameras;
FIG. 2 is a diagrammatical top view of an embodiment of the scanning device, especially showing three pairs of cameras, the cameras of each pair being symmetrically placed on both sides of a scanning area, with dedicated horizontal angular orientations of the cameras;
FIG. 3 is a diagrammatical front view of an embodiment of the scanning device including a path, especially showing dedicated vertical orientations of the cameras of the second tripod at the output of the scanning area, and also the specific scopes of such front cameras;
FIG. 4 is a diagrammatical lateral view of an embodiment of the scanning device, with a calibration target placed inside the scanning area directly on the ground and extending along a median longitudinal plane;
FIG. 5 is a diagrammatical lateral view of an embodiment of the scanning device, including a path along the scanning area; and
FIG. 6 is a diagrammatical view of an architecture of the scanning system, especially showing the conversion module and the extracting module.

### Description of Embodiments

The invention relates to the technical field of animal husbandry and breeding.

As aforementioned, the invention is directed for recording measurements of animals, especially of quadruped animals.

Such animals can be of any species, especially livestock animals such as sheep and cattle (*i.e*. oxen and cows), goats, eventually deer, dogs and cats.

Preferably, the animals 1 can be horses 1, especially race horses 1.

So in the examples shown in the figures, the diagrammatically represented animal is a horse 1 in a non-limitative manner.

Thus, the invention first relates to a scanning device 2 for recording quadruped animal measurements.

According to the invention, the scanning device 2 comprises at least two frames 3. Each of the two frames 3 is located on a right side and on a left side. Moreover, the two frames 3 are parallel and symmetrically spaced from each other.

The scanning device 2 also comprises a scanning area 4 defined between the at least two frames 3. The scanning area 4 is dimensioned to allow an animal inside. Especially, the scanning area 4 is long and wide enough to receive an entire horse.

For example, the scanning area 4 can be up to four meters long and up to three meters wide.

Thus, the scanning area 4 is laterally surrounded by the two frames 3, like a corridor. Thus, the scanning area 4 comprises an entry 40 at an end and an output 41 at the opposite end, so the horse 1 can enter and exit the scanning area 4, following a longitudinal direction of movement from the entry 40 to the output 41.

According to an embodiment, the scanning device 2 can be placed and/or integrated into a covered place, such as a building or a tent, or into a vehicle, such as a horse trailer.

According to an embodiment, the scanning device 2 is directly placed on the ground. So the scanning area 4 is directly delimited on the ground. The scanning device 2 can also be, at least in part, placed above the passage of the animal.

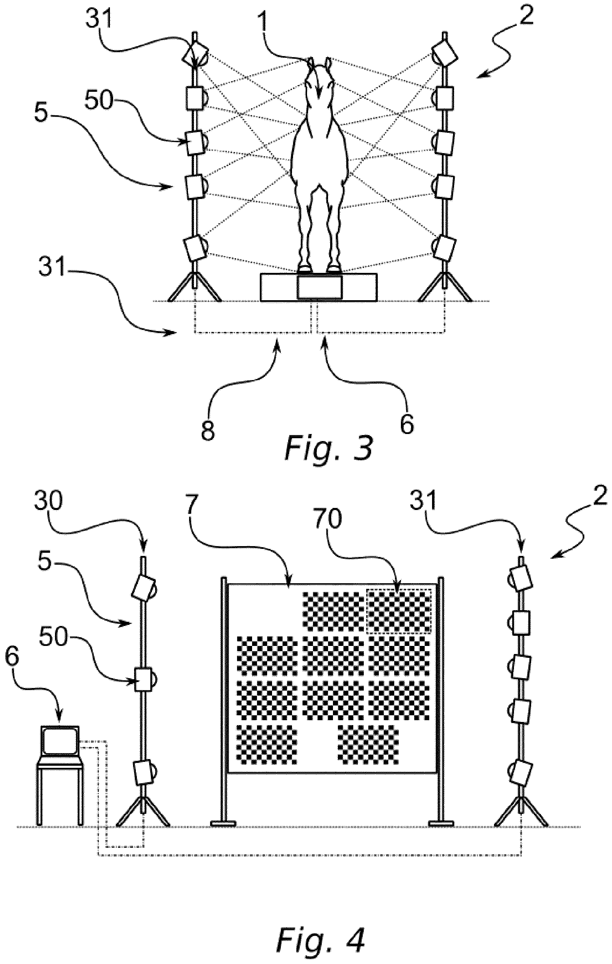

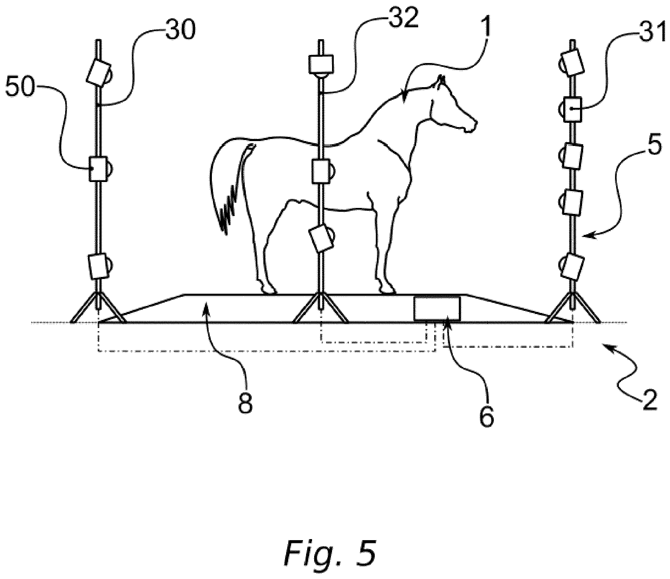

According to another embodiment, such as shown in the figures 3 and 5, the scanning area 4 comprises a path 8, on which the horse 1 can walk. Such path 8 is like a platform, raised compared to the floor level, with inclined ends to allow the horse 1 to walk in and out of the scanning area 4, without frightening it.

According to another embodiment, the scanning area 4 can be delimited on the ground, directly or through any material, such as a board or a tarp.

According to an embodiment, the path 8 has a maximum height of 40 cm.

The scanning device 2 further comprises a scanning means 5 with at least two cameras 50. Said cameras 50 are of a digital type, especially digital recording image type and/or video type.

Moreover, the cameras 50 are a type of depth camera.

According to an embodiment, the cameras 50 have at least an internal sensor of a color depth RGB-D type (for « Red Green Blue - Depth » type). Such an RGB-D type camera 50 combines depth sensors to the conventional image captured by the RGB camera sensor, so that related distance information is recorded into the digital image file, especially for each pixel of such a digital image file.

According to an embodiment, the cameras 50 have captured wavelengths close to infrared light. Preferably, the wavelengths of the cameras are about 850 nanometers. The specific wavelengths of the cameras 50 allow shooting images/videos in light and dark environments without additional lights, so the scanning device 2 can be used inside and/or outside in any brightness conditions. Infrared wavelengths also better allow capturing depth, even with shadows and a monochrome and uniform color of the fur of the animal.

According to an embodiment, the shooting frequency of capture of the cameras 50 is about 30 Hz (Hertz), so that approximately 30 images per second are captured by each camera 50. Such high shooting frequency allows many images to be taken so that they can be transcribed into a video and capture the motions of the animal. Moreover, such high shooting frequency allows many pictures to be taken, so that some of them can be sorted and/or removed, if the animal is moving and the picture is blurred or not well defined. Also, such high shooting frequency allows selection of all images captured at the same time, depending on the position of the animal and/or if some of them are blurred.

Moreover, each of the at least two cameras 50 is mounted on each of the at least two frames 3, on the right side and the left side. In other words, one of the at least two cameras 50 is mounted on the right of the two frames 3, while the other is mounted on the left of the frames 3.

Also, each of the at least two cameras 50 has an acquisition scope orientated toward the scanning area 4. Thus, when recording, the cameras 50 capture images and/or video of the animal located inside the scanning area 4, in a stereoscopic way.

The scanning device 2 further comprises a digital storage means 6 of the images and/or the videos captured by the at least two cameras 50. Such storage means can be of any type, especially a computer including a memory.

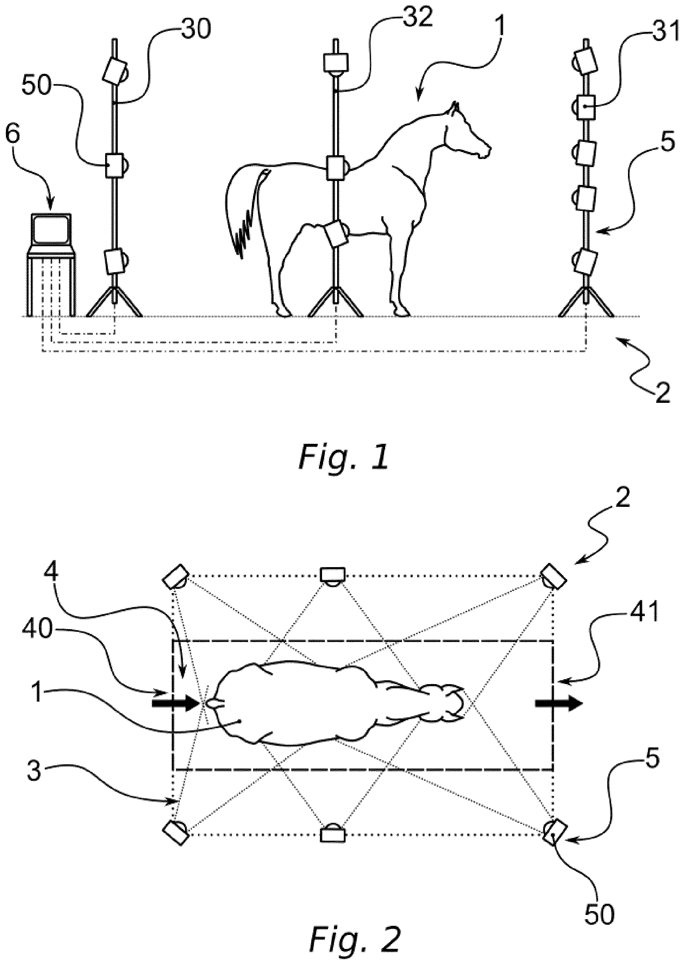

According to an embodiment, the storage means 6 can be placed on the ground, near the scanning area 4, such as shown in figures 1 and 4, or above the passage of the animal.

According to another embodiment, as shown in figures 3 and 5, the storage means 6 can be placed under the path 8, so that all the cameras 50 can be directly linked with shorter wires to the centrally positioned storage means 6. Also, located under the path 8, the storage means 6 and the wires are protected from the animal when going through the scanning area 4.

According to the invention, the scanning means 5 comprise at least six cameras 50 grouped in three pairs, each of the three pairs of the at least six cameras 50 being symmetrically mounted on the at least two frames 3. In other words, one of the two cameras 50 of one of the three pairs is located on the right of the frames 3, while the other camera 50 of said pair is located on the left frame 3. Moreover, the symmetry is along a longitudinal median axis extending from the entry 40 to the output 41.

According to an embodiment, each of the at least two frames 3 comprises a first extremity and an opposite extremity. The first extremity is located at or close to the entry 40 and the other opposite extremity is located at or close to the output 41.

Moreover, the frames 3 comprise tripods on which the cameras 50 are attached. Preferably, each of the at least two frames 3 comprises a first tripod 30 at the first extremity, a second tripod 31 at the opposite extremity and a middle tripod 32, located between the first tripod 30 and the second tripod 31.

According to an embodiment, several middle tripods 32 can be positioned between the first rear tripod 30 and the second front tripod 31, along the scanning area 4.

Moreover, the tripods 30,31,32 are located around the scanning area 4, so on both sides of the scanning device 2, along each of the frames 3. Such lateral positions of the tripods 30,31,32 create a corridor without any visible obstacle in front of the animal, so that it is not frightened or stressed when passing through the scanning device 2, also avoiding accident risks that can hurt the animal, the operators and the materials of the scanning device 2.

The at least six cameras 50 are mounted on the corresponding first tripod 30, second tripod 31 and middle tripod 32.

According to an embodiment, the tripods 30,31,32 are telescopic.

According to an embodiment, the tripods 30,31,32 have a height up to 3 m, preferably about 2 m +/- 50 cm.

A first embodiment with three pairs of the six cameras 50 is shown in figure 2, wherein the scope of each of the cameras 50 is orientated toward the center of the scanning area 4, in an angular configuration to cross the longitudinal median axis.

In another embodiment with also three pairs of the six cameras 50, not shown, wherein the scopes of the cameras 50 are orientated toward the center of the scanning area 4, in a parallel or almost parallel direction (*i.e*. orthogonally to the longitudinal median plane).

According to another embodiment, the scanning means 5 comprise at least eight cameras 50 grouped in four pairs. Moreover, each of the cameras of the four pairs of the at least eight cameras 50 is symmetrically mounted on the at least two frames 3. In other words, for each of the eight pairs, one camera 50 is located on the right side, while the other is located on the left side.

Also, as there are only three tripods 30,31,32 and four pairs of cameras 50, one of the tripods 30,31,32 can receive several cameras 50, vertically mounted at different heights. Thus, two cameras 50 of one of the pairs are located at the same height on each of the two frames 3.

According to a preferred embodiment, such as shown in figure 4, the scanning means 5 comprise at least twenty-two cameras 50 grouped in eleven pairs, each of the eleven pairs of the at least twenty-two cameras being symmetrically mounted on the at least two frames 3.

According to a specific embodiment, especially for capturing a horse 1, the second tripod 31 of each of the at least two frames 3 comprises five of the at least twenty-two cameras 50. As aforementioned, the five cameras 50 are vertically located at different first heights along the second tripod 31.

Moreover, the second tripod 31 of each of the at least two frames 3 comprises three of the at least twenty-two cameras 50, which are also vertically located at different second heights along the second tripod 31. Finally, the middle tripod 32 of each of the at least two frames 3 comprises three of the at least twenty-two cameras 50, which are also vertically located at different middle heights along the middle tripod 32.

According to an embodiment, the lower cameras 50 are positioned on the corresponding tripods 30,31,32 at a height of between 10 and 40 cm from the ground, preferably at a height comprised between 10 and 40 cm from the top surface of the path 8, preferably at a height of 25 cm.

According to an embodiment, each of the cameras 50 is angularly orientated relative to the scanning area 4.

Firstly, the cameras 50 have dedicated vertical angular orientations. In other words, each of the cameras 50 is angularly orientated relative to a vertical axis, especially relative to the vertical frame of the corresponding tripods 30,31,32. Thus, the scope of each of the cameras 50 is orientated to the top, to the bottom or horizontally (or almost horizontally), toward the scanning area 4 (*i.e.* crossing the longitudinal median plane).

Moreover, the vertical angular orientation of each of the cameras 50 depends on the position on the corresponding tripods 30,31,32. Lower cameras 50 are angularly orientated to the top, in order to capture part of the lower body of the animal, especially the inner legs and the belly. Top cameras 50 are angularly orientated to the bottom, in order to capture the upper body of the animal, especially the back and the spine. Middle cameras 50 are angularly orientated horizontally or almost horizontally, in order to capture part of the corresponding front and rear body of the animal, especially the rear, the face and the chest.

An example of vertical angular orientations of the cameras 50 is shown in figure 1.

Also the cameras 50 have dedicated horizontal angular orientations. In other words, each of the cameras 50 is angularly orientated relative to a horizontal plane, especially a horizontal plane orthogonally crossing the vertical frame of the corresponding tripods 30,31,32. Thus, the scope of each of the cameras 50 is orientated to the right or to the left, clockwise or counterclockwise, towards the scanning area 4.

Moreover, the horizontal angular orientation of each of the cameras 50 depends on the position of the corresponding tripods 30,31,32. Rear cameras 50 on the first tripod 30 are angularly orientated to capture the rear body of the animal, especially the inner legs, preferably the opposite leg and both sides of the rear and back. Front cameras 50 on the second tripod 31 are angularly orientated to capture the front part of the animal, especially the inner legs, preferably the opposite leg, and also the face, the chest and both sides of these parts. The middle cameras 50 on the middle tripod 32 are angularly positioned to capture the main body of the animal, especially the back, the belly and the spine.

An example of horizontal angular orientations of the cameras 50 is shown in figure 2.

Vertical and horizontal angular orientations of the cameras 50 allow an entire recording of all of the animal body parts, avoiding any occultation of some of the body compared to an orthogonal orientation of the scopes of the cameras 50 (for example the inner part of the opposite leg). Especially when capturing a horse-type animal, the inclined vertical and horizontal orientation is optimized due to the positioning of the horse 1 in a « square position » (*i.e.* each of the legs being straight).

According to an embodiment, as aforementioned, the at least two frames 3 are spaced managing the entry 40 of the scanning area 4 at the first end and the output 41 of the scanning area 4 at the opposite end, defining a corridor between the at least two frames 3. Also, the scanning area 4 is dimensioned to allow an animal, especially a horse, to move along the corridor, crossing the scanning area 4 through the entry 40 to the output 41.

According to an embodiment, the at least two frames 3 are spaced from borders of the scanning area 4, so that each of the cameras 50 is spaced with a lateral distance from the median longitudinal plane of the scanning area 4. Moreover, the lateral distance is from 60 cm (centimeters) to 120 cm.

According to a preferred embodiment, the lateral distance is about 80 cm.

Such distance can be determined depending on the size of the animal to scan, and also the characteristics of the cameras 50, for example, the scope of the cameras 50.

According to an embodiment, once the cameras 50 are mounted on the frames 3, it is necessary to calibrate the cameras 50, depending on the animal to be scanned, the characteristics of the cameras 50, and also the orientation of their scope.

Therefore, the scanning device 2 comprises a calibration target 7. Moreover, the calibration target 7 is a black and white checkered board. The calibration target 7 is also movable, for example mounted on a light movable frame, for example, a metal frame on which a canvas is fixed.

The calibration target 7 is placed into the scanning area 4, in front of the at least six cameras 50, specifically crossing the scopes of all of the at least six cameras 50. So the calibration target 7 is dimensioned to be placed inside the scanning area 4, without any animal inside, so that the scopes of all the cameras 50 capture at least a part of the calibration target 7.

According to the invention, the calibration target 7 is flat or has a plane surface, such as a plate and preferably a canvas, of small thickness, for example less than 5 mm thick, preferably less than 2 mm.

According to an embodiment, the calibration target 7 is positioned and extends along the median longitudinal plane of the scanning area 4.

It is to be noticed that the calibration target 7 is checkered, with square or round black and white adjacent tags or dots, regularly located all over each of the two sides of the board of the calibration target 7.

According to an embodiment, the calibration target 7 comprises square areas, such as checkered squares 70, distributed on each side of the two surfaces.

According to an embodiment, the calibration target 7 comprises about 120 tags or dots, especially on each side of the canvas (*i.e.* recto and verso). The canvas is opaque (*i.e.* non-translucent), so that tags or dots on one side are not visible from the other side.

According to an embodiment, the printing process of the checkered calibration target 7 is dedicated to the type of the cameras 50.

According to an embodiment, the calibration target 7 material is dedicated to the type of the cameras 50. For example, the calibration target 7 material can be a matte material (*i.e.* non reflective).

According to an embodiment, the calibration target 7 is configured and dedicated to a species of animal, especially some characteristics of one of the species, like the color, the size, *etc.*

According to an embodiment, the calibration target 7 is dedicated to a horse-type animal. Such a calibration target 7 comprises several identical checkered squares 70, positioned in a horse-like shape or similar to a horse-like silhouette. Moreover, one of the checkered squares 70 is used for calibration of each of the cameras 50, an entire or a part of one of the checkered squares 70 can be used for calibration of more than one camera 50. An example of such a calibration target 7 with a horse-type configuration of the checkered squares 70 is represented in figure 4.

According to an embodiment, the cameras 50 are configured to simultaneously capture the images and/or videos. Preferably, the cameras 50 can be linked and controlled in a stereo active vision manner. Such stereo active vision manner comprises at least a grid, allowing a position software treatment of the coordinates of the points inside the grid, such as a triangulation treatment of the coordinates into one or more repositories, especially a global repository, while passive stereo active vision manner without a grid cannot handle non-textured images, which is mostly the case when capturing images of the fur of an animal.

According to an embodiment, the cameras 50 are configured to capture the images and/or videos at a same dedicated frequency, preferably at a frequency of 30 Hertz (*i.e.* 30 images per second). Such a frequency allows capturing several images of an animal by the cameras 50, even if the animal moves.

The invention also relates to a scanning system 10 for recording quadruped animal measurements.

According to the invention, the scanning system 10 comprises the scanning device 2, such as described above.

Moreover, the scanning system comprises a software being installed on and executed by a computer terminal 11 linked to the digital storage means 6.

Such software is implemented for computing data 12, which is digital and comprising at least the images and/or the videos captured by the at least six cameras 50 of the scanning means 5. Such data 12 can be of any type, such as digital files of an image and/or video type.

According to the invention, the software comprises a conversion module 13 of the data 12, the conversion module transforms the data 12 into a tri-dimensional point cloud 14, also called "cloud 14 of points".

Such a point cloud 14 is a set of points in a global repository, such as a tri-dimensional cartesian frame reference with three coordinates (e.g. X, Y, Z). Moreover, the point cloud 14 does not comprise topology or any organized pattern of the points (for example, compared to a point mesh), so that the point coordinates of the cloud 14 are the closest to the real dimensions captured and recorded by the cameras 50.

Moreover, the point cloud 14 allows to get a high density of points, compared to known interpolation software treatments. Thus, the invention keeps as close to reality the high numbers of points captured by the photographic sensors of each of the cameras 50. Additionally, a point cloud 14 is faster to record and to treat via software, in particular for a high number of data (*e.g.* more than 1 million).

According to an embodiment, the conversion module 13 transforms at least one depth map of the points into the point cloud 14. Especially several depth maps are converted. Such depth maps are obtained through the depth sensors of the cameras 50.

Thus, the successive depth maps are transformed into depth levels to generate the 3D point cloud 14.

According to an embodiment, the conversion module 13 comprises at least one filter, the filter being applied to at least one depth map of points.

Many filters can be applied, in order to optimize recording quality and correct any recording error, especially due to a Gaussian distribution noise when recording depths.

For example, a filter can remove outlines and/or a bilinear filter can smooth the data without modifying the outlines. Also, an average filter can be applied on each of the depth maps captured during a period, in order to compensate or offset the coordinate differences between successive recordings, for example if the animal was moving.

Moreover, on the final point cloud 14, other software filters can be applied, one or several times, in order to improve the quality of the points of the cloud 14. Preferably an average is made on ten successive depth maps.

For example, a Voxel-Grid filter can be applied once or more.

According to the invention, the software comprises an extracting module 15 of the animal measurements from the point cloud 14.

Such extraction is operated by specific landmarks applied on some points of the cloud 14. Such landmarks depend on the animal type.

For example, for a horse-type animal, the landmarks can be, in a non-exhaustive manner: tip of a horse muzzle, head base, horse withers, top of the rump, sternum, shoulder point, point of the buttocks, ventral iliac spine of the pelvis, dorsal iliac spine of the pelvis, hip joint, knee, hock, back and front feet, fetlock, and/or elbow.

According to an embodiment, the landmarks can be manually selected by an operator among the points of the point cloud 14. According to another embodiment, the landmarks are automatically selected among the points of the point cloud 14, through a tridimensional surface software treatment, such as a machine learning method, especially a deep-learning method of the type of self-learning or of self-supervised learning.

Moreover, from the landmarks, it is possible to calculate anatomic distances and animal dimensions.

For example, for a horse-type animal, the anatomic distances can be, in a non-exhaustive manner: height at withers, height at the rump, horse length (from body to head), chest width, pelvis width, leg circumferences and/or trunk circumferences (thoracic and/or abdominal).

Then, the animal measurements can be recorded into a dedicated measurements file 16, for example a spreadsheet file.

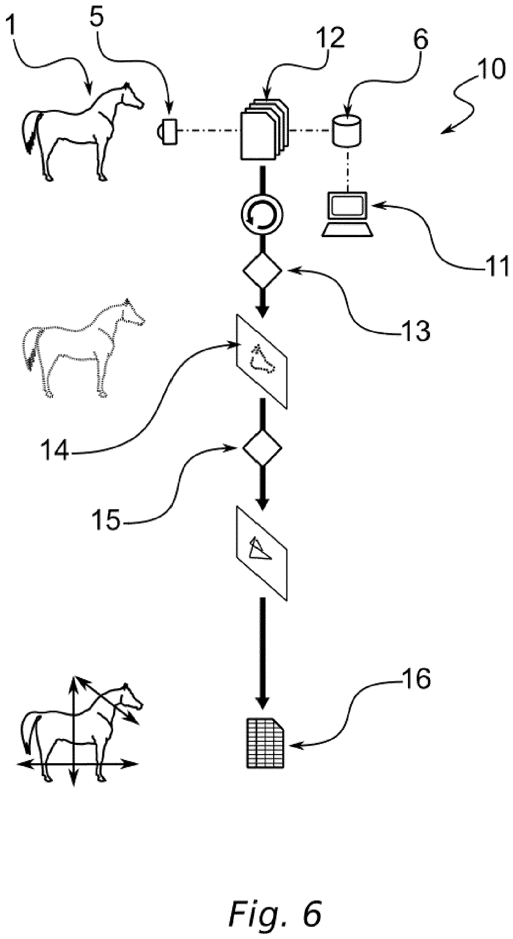

An example of a simplified architecture of the scanning system 10 is shown in figure 6.

The invention also relates to a scanning method for recording quadruped animal measurements.

According to the invention, the scanning method is dedicated to the use of the scanning device 2.

According to an embodiment, the scanning method is also dedicated to execute the scanning system 10.

First the scanning method comprises a calibration step of the cameras 50. Therefore, the scanning method consists in positioning the calibration target 7 along the median longitudinal plane of the scanning area 4 to calibrate the cameras 50 on checkered squares 70 of the calibration target 7. Thus, the cameras 50 capture the calibration target 7, especially the checkered squares whereupon the scope of each of the cameras 50 is orientated.

It is to be noticed that the vertical and horizontal orientations of the cameras 50 is adjusted in order to focus on one or several checkered squares 70. The calibration of the cameras 50 is performed by detecting the position of at least one of the corners of one of the checkered squares 70 captured by each of the cameras 50. As each of the cameras 50 has a depth sensor and its own referential, each of the detected corners has tridimensional coordinates into the own referential of each of the cameras 50.

It is to be noticed that the detection of the corners of the checkered squares 70 can be realized in a sweeping manner, for example from top left to bottom right, line per line, of each of the squares of the checkered squares 70.

Then, a global repository of all the cameras 50 relative to each other is calculated, for each of the cameras 50, using at least one of the detected positions of the corner. Such calculating is possible because the own referential of each of the cameras 50 is known, due to the positions of the corresponding tripods 30,31,32, and also the position of the calibration target 7 and of all of its checkered squares 70.

It is to be noticed that the calculations of the global repository are obtained through an automatic software module. For example, such a module can comprise a mathematic algorithm, such as a geometrical transformation, in order to obtain as a result an extrinsic matrix of the calibration.

Finally, the calibration target is removed from the scanning area 4.

Then, once the calibration has been completed, the scanning method comprises a recording of the animal by the cameras 50 and a conversion and an extraction to record the animal measurements.

Therefore, an animal is placed inside the scanning area 4 and images and/or videos of the animal are captured with all of the cameras 50.

As aforementioned, the recording is synchronized, so that all the cameras 50 operate at the same time. Especially considering the high frequency of 30 Hz, all the cameras 50 record in the same time lapse of 1/30 second.

According to the invention, the captured images and/or videos are stored as digital data 12, especially in the storage means 6.

Then, the digital data 12 are converted into a tri-dimensional point cloud 14 into the global repository.

Finally, the animal measurements are extracted from the point cloud 14.

As aforementioned, the extraction of the measurements can be obtained by calculations from the landmarks.

## Claims

1. A scanning device (2) for recording quadruped animal measurements, comprising
- at least two frames (3), each being located on a right side and on a left side, being parallel and symmetrically spaced from each other,
- a scanning area (4) defined between the at least two frames (3), the scanning area (4) being dimensioned to allow an animal (1) inside,
- a scanning means (5), comprising at least two cameras (50) being of an image type and/or of a video type,
i) each of the at least two cameras (50) being mounted on each of the at least two frames (3), on the right side and the left side,
ii) each of the at least two cameras (50) having an acquisition scope orientated toward the scanning area (4),
- a digital storage means (6) for storing the images and/or the videos captured by the at least two cameras (50),
**characterized in that**
- the cameras (50) being of a type of depth camera,
and **in that**
the scanning means (5) comprise
- at least six cameras (50) grouped in three pairs, each of the three pairs of the at least six cameras (50) being symmetrically mounted on the at least two frames (3).

2. The scanning device (2) according to claim 1, **characterized in that** each of the at least two frames (3) comprises
- a first extremity and an opposite extremity,
- a first tripod (30) at the first extremity,
- a second tripod (31) at the opposite extremity,
- a middle tripod (32), located between the first tripod (30) and the second tripod (31), the at least six cameras (50) being mounted on the corresponding first tripod (30), second tripod (31) and middle tripod (32).

3. The scanning device (2) according to the preceding claim, **characterized in that** the scanning means (5) comprise at least eight cameras (50) grouped in four pairs, each of the four pairs of the at least eight cameras (50) being symmetrically mounted on the at least two frames (3).

4. The scanning device (2) according to the preceding claim, **characterized in that** the scanning means (5) comprise at least twenty-two cameras (50) grouped in eleven pairs, each of the eleven pairs of the at least twenty-two cameras (50) being symmetrically mounted on the at least two frames (3).

5. The scanning device (2) according to the preceding claim, **characterized in that**
- the first tripod (30) of each of the at least two frames (3) comprises five of the at least twenty-two cameras (50), vertically located at different first heights along the first tripod (30),
- the second tripod (31) of each of the at least two frames comprises three of the at least twenty-two cameras (50), vertically located at different second heights along the second tripod (31),
- the middle tripod (32) of each of the at least two frames comprises three of the at least twenty-two cameras (50), vertically located at different middle heights along the middle tripod (32).

6. The scanning device (2) according to any of the preceding claims, **characterized in that** the at least two frames (3) being spaced from borders of the scanning area (4), so that each of the cameras (50) is spaced with a lateral distance from a median longitudinal plane of the scanning area (4), the lateral distance being from 60 cm to 120 cm.

7. The scanning device (2) according to the preceding claim, **characterized in that** the lateral distance is approximately 80 cm, preferably at a maximum of 80 cm.

8. The scanning device (2) according to any of the preceding claims, **characterized in that**
- the at least two frames (3) are spaced managing an entry (40) of the scanning area (4) at a first end and an output (41) of the scanning area at an opposite end, defining a corridor between the at least two frames (3),
- the scanning area (4) is dimensioned to allow an animal (1) to move along the corridor, crossing the scanning area (4) through the entry (40) to the output (41).

9. The scanning device (2) according to any of the preceding claims, **characterized in that** it further comprises a calibration target (7), the calibration target (7) being a black and white checkered board, movable to be placed in the scanning area (4) along a median longitudinal plane, in front of the at least six cameras (50).

10. The scanning device (2) according to any of the preceding claims, **characterized in that** the at least six cameras (50) have capture wavelengths close to infrared light, preferably capture wavelengths about 850 nanometers.

11. The scanning device (2) according to any of the preceding claims, **characterized in that** each of the at least six cameras (50) of being a type of depth camera having at least an internal sensor of a color depth RGB-D type.

12. A scanning system (10) for recording quadruped animal measurements, comprising
- the scanning device (2) according to any of the preceding claims,
- software computing data, the software being installed on and executed by a computer terminal (11) linked to the digital storage means (6), the data being digital and comprising at least the images and/or the videos captured by the at least six cameras (50),
wherein
- the software comprises a conversion module (13) of the data, the conversion module (13) transforming the data into a tri-dimensional point cloud (14), **characterized in that**
- the software comprises an extracting module (15) of the animal measurements from the point cloud (14).

13. The scanning system (10) according to the preceding claim, **characterized in that** the conversion module (13) transforms at least one depth map of the points into the point cloud (14).

14. The scanning system (10) according to the preceding claim, **characterized in that** the conversion module (13) comprises at least one filter, the filter being applied to the at least one depth map of points.

15. A scanning method for recording quadruped animal measurements, using the scanning device (2) according to claim 9, comprising the steps of
- positioning the calibration target (7) along the median longitudinal plane of the scanning area (4),
- calibrating the cameras (50) on checkered squares (70) of the calibration target (7),
- removing the calibration target (7),
- placing an animal (1) inside the scanning area (4),
- capturing animal images and/or videos with all of the cameras (50),
**characterized in that** it comprises the steps of
- calibrating the cameras (50) by detecting the position of at least one corner of one of the checkered squares (70) captured by each of the cameras (50),
- calculating a global repository of all the cameras (50) relative to each other, regarding the at least one of the corners' detected position,
and **in that**
- storing captured images and/or videos into digital data,
- converting the digital data into a tri-dimensional point cloud (14) in the global repository,
- extracting the animal measurements from the point cloud (14).
